# EUROPEAN PATENT APPLICATION

(11) **EP 2 133 101 A1**
(43) Date of publication of application: **16.12.2009**
(21) Application number: 08739009.2
(22) Date of filing: 27.03.2008
(51) Int. Cl.: A61L 2/20, A61L 2/22, B01L 1/00

(54) **SYSTEM FOR MAINTAINING STERILE ENVIRONMENT**

(30) Priority: 28.03.2007 JP 2007084959; 27.02.2008 JP 2008045876
(71) Applicant: Sanyo Electric Co., Ltd., Osaka 570-8677 (JP)
(72) Inventor: ONISHI, Jiro, Ota-shi Gunma 373-0819 (JP); YOKOI, Yasuhiko, Ota-shi Gunma 373-0816 (JP); IWAMA, Akifumi, Tsukuba-shi Ibaraki 305-0861 (JP)
(74) Representative: Holmes, Matthew Peter
(86) International application number: PCT/JP2008/055880
(87) International publication number: WO 2008/123359

(57) **Abstract**

Provided is a sterile environment maintaining apparatus capable of sterilizing a sterile room in a shorter time than conventional ones.

The sterile environment maintaining apparatus according to the present invention comprises a sterile room 10 having an inlet opening 11 and an outlet opening 12, a SEPA filter 3 placed so as to cover the inlet opening 11 or the inlet opening 11 and the outlet opening 12 of the sterile room 10, and a sterilizing material supplying part supplying a sterilizing material such as hydrogen peroxide to the sterile room 10, and the sterile room 10 is provided with a detoxifying material supplying part spraying a detoxifying material such as ozone toward the HEPA filter 3.

## Description

### FIELD OF THE INVENTION

The present invention relates to a sterile environment maintaining apparatus capable of maintaining sterile environment in a space within the apparatus such as isolator and clean bench.

### DESCRIPTION OF RELATED ART

The sterile environment is, in operating a work, an environment illimitably close to dust-free biological cleanness for avoiding mixture with a thing other than material required for the work. A sterile work station is an apparatus for operation of a work in a sterile room maintained in sterile environment in a sterile environment maintaining apparatus, including, as representative examples, an isolator, a clean bench, a safety cabinet and the like for cell preparation.

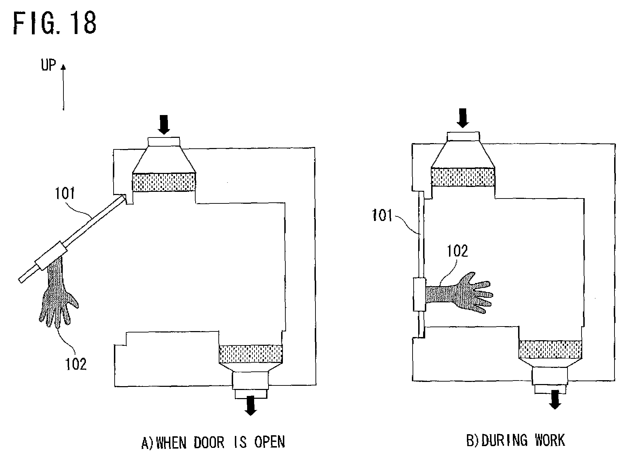

The isolator is a sterile work station which allows a work in the sterile room while maintaining physical isolation of the sterile room from the surrounding environment. As an example shown in FIG. 18, the isolator has a structure capable of maintaining the isolated state of the sterile room by a work through a glove 102 (work means allowing the work in the sterile room) with a front door 101 closed.

The clean bench is a sterile work station which maintains the sterile state of the sterile room by means of air current control in the sterile room. Even if a front door of the sterile room is in a half open state (work means allowing the work in the sterile room) during the work, the sterile state is maintained due to the air current.

The safety cabinet is a sterile work station which maintains the sterile state of the sterile room by means of air current control in the sterile room. Even if the front door of the sterile room is in a half open state (work means allowing the work in the sterile room) during the work, the sterile state is maintained due to the air current. Further, the air current is controlled not to spread the material in the sterile room.

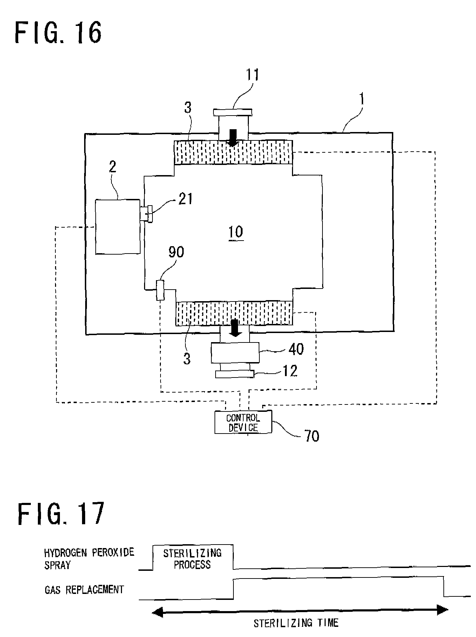

Conventionally, in the isolator, as shown in FIG. 16, a sterile room 7.0 having an inlet opening 11 and an outlet opening 12 is formed in a cabinet 1. In the sterile room 10, HEPA filters 3, 3 whose object is capturing fine particles are placed so as to cover the inlet opening 11 and the outlet opening 12 each.

Also, to the sterile room 10, connected is a hydrogen peroxide supplying tube 21 (sterilizing material supplying part) for supplying hydrogen peroxide which is a sterilizing material from a hydrogen peroxide generator 2 (sterilizing material generating part). A hydrogen peroxide, eliminating filter 40 is placed at a position adjacent to the outlet opening 12.

Further, the sterile room 10 is provided with a sensor unit 90 detecting temperature, humidity, hydrogen peroxide concentration and the like. A detection signal is supplied to a control device 70, and the hydrogen peroxide generator 2 and the like are controlled by the control device 70.

In the isolator described above, after one work in the sterile room 10 ends, for a next work, hydrogen peroxide gas is sprayed in the sterile room 10 from the hydrogen peroxide generator 2 to fill the sterile room 10 with the hydrogen peroxide gas for sterilization of the sterile room 10 (for example, See Japanese Laid-Open Patent Publication No. 2002-360672).

And then, after the sterilization process, performed is a gas replacement process in which the hydrogen peroxide gas in the sterile room 10 is replaced with air by taking in air from the inlet opening 11 via the HEPA filter 3 while eliminating the hydrogen peroxide gas in the sterile room 10 via the HEPA filter 3 from the outlet opening 12.

### DISCLOSURE OF THE INTENTION

### PROBLEMS TO BE SOLVED BY THE INVENTION

However, in the conventional isolator, as shown in FIG. 17, it is necessary to perform the gas replacement process for a long time after the sterilization process of spraying the hydrogen peroxide gas, and therefore, it is problematic that time required for the sterilization of the sterile room 10 is very long.

An object of the present invention is to provide a sterile environment maintaining apparatus capable of achieving sterilization of the sterile room in a shorter time.

### MEANS FOR SOLVING THE PROBLEM

As the results of intensive studies to achieve the above mentioned object, the inventors of the present invention found that a factor of taking long time for the gas replacement process after the sterilization process in the conventional isolator is that filling of the sterile room with the hydrogen peroxide gas in the sterilization process results in attachment of hydrogen peroxide to the HEPA filter in liquid state, and it is difficult to detach hydrogen peroxide from the HEPA filter in the gas replacement. And they completed the present invention.

A first sterile environment maintaining apparatus according to the present invention comprises a sterile room having an inlet opening and an outlet opening, a fine particle capture filter placed so as to cover the inlet opening or the inlet opening and the outlet opening of the sterile room, and a sterilizing material supplying part supplying a sterilizing material to the sterile room, and is provided with a detoxifying material supplying part releasing a detoxifying material for detoxifying the sterilizing material toward the fine particle capture filter.

It is preferable that the detoxifying material supplying part releases the detoxifying material toward an inlet opening side surface of the fine particle capture filter placed so as to cover the inlet opening.

The sterilizing material supplying part supplies gas or liquid fine particle including the sterilizing material to the sterile room. The detoxifying material supplying part sprays the gas or liquid fine particle including the detoxifying material toward the fine particle capture filter.

Here, the sterilizing material is a first active oxygen species, and the detoxifying material is a second active oxygen species. Also, in the case where the sterilizing material is hydrogen peroxide or ozone, the detoxifying material may be alkaline water.

According to the sterile environment maintaining apparatus described above, the detoxifying material is sprayed directly to the fine particle capture filter to which the sterilizing material is attached in the sterilization process, and therefore, the sterilizing material on the fine particle capture filter is effectively decomposed by the detoxifying material, and detoxified in a short time.

A second sterile environment maintaining apparatus according to the present invention comprises a sterile room having an inlet opening and an outlet opening, a fine particle capture filter placed so as to cover the inlet opening or the inlet opening and the outlet opening of the sterile room, and a sterilizing material supplying part supplying a sterilizing material to the sterile room, and is provided with irradiating means for detoxifying the sterilizing material toward the fine particle capture filter.

Here, the sterilizing material is an active oxygen species, and the irradiating means is either of an ultraviolet irradiation device, an ultrasound irradiation device, a heat irradiation device or an infrared irradiation device, or any combination thereof.

According to the sterile environment maintaining apparatus described above, either of the ultraviolet, ultrasound, or infrared, or any combination thereof is irradiated directly to the fine particle capture filter to which the sterilizing material is attached in the sterilization process, and therefore, the sterilizing material on the fine particle capture filter is effectively decomposed by the detoxifying material, and detoxified in a short time.

A third sterile environment maintaining apparatus according to the present invention comprises a sterile room having an inlet opening and an outlet opening, a fine particle capture filter placed so as to cover the inlet opening or the inlet opening and the outlet opening of the sterile room, and a sterilizing material supplying part supplying a sterilizing material to the sterile room, and the fine particle capture filter includes a detoxifying material for detoxifying the sterilizing material.

According to the sterile environment maintaining apparatus described above, the fine particle capture filter to which the sterilizing material is attached in the sterilization process includes the detoxifying material for detoxifying the sterilizing material, and therefore, the sterilizing material reacts with the detoxifying material, and is detoxified in a short time.

Also, according to the sterile work station such as an isolator using the sterile environment maintaining apparatus of the present invention described above, it is possible to perform detoxification in a shorter time and perform the work in the sterile room effectively.

### EFFECT OF THE INVENTION

According to the sterile environment maintaining apparatus of the present invention, it is possible to sterilize the sterile room in a shorter time.

### BRIEF DESCRIPTION OF THE DRAWINGS

[FIG. 1] FIG. 1 is a view showing a structure of a first isolator of the present invention;
[FIG. 2] FIG. 2 is a perspective view of an ozone spraying device;
[FIG. 3] FIG. 3 is a side view of the ozone spraying device;
[FIG. 4] FIG. 4 is a front view of the ozone spraying device;
[FIG. 5] FIG. 5 is a plain view of the ozone spraying device;
[FIG. 6] FIG. 6 is a view showing a structure of an ozone generator;
[FIG. 7] FIG. 7 is a view showing another structure of the ozone generator;
[FIG. 8] FIG. 8 is a view showing a structure of a hydrogen peroxide generator;
[FIG. 9] FIGS. 9 are views showing other structures of the hydrogen peroxide generator;
[FIG. 10] FIGS. 10 are views showing a plurality of examples of a sterilization process in the isolator;
[FIG. 11] FIG. 11 is a view showing a structure of a second isolator of the present invention;
[FIG. 12] FIG. 12 is a front view showing an arrange of a UV lamp;
[FIG. 13] FIG. 13 is a plain view showing the UV lamp and a reciprocating transfer mechanism;
[FIG. 14] FIG. 14 is a view showing one example of the sterilization process in the isolator;
[FIG. 15] FIGS. 15 are views showing reaction chemical formulas of a detoxifying process;
[FIG. 16] FIG. 16 is a view showing a structure of a conventional or a third isolator of the present invention;
[FIG. 17] FIG. 17 is a view showing a conventional sterilizing process;
[FIG. 18] FIGS. 18 are cross-sectional views of the isolator shown in FIG. 16, with a central part thereof cut in an up and down direction, taken from a direction perpendicular to a plane of paper.
[FIG. 19] FIG. 19 is a view showing a structure of the first isolator of circulation sterilization type; and
[FIG. 20] FIG. 20 is a view showing a structure of the first isolator with an HEPA filter placed only at an inlet opening.

### EXPLANATION OF REFERENCES

1. Cabinet
10. Sterile room
11. Inlet opening
12. Outlet opening
2. Hydrogen peroxide generator
29. Flow switching bulb
3. HEPA filter
4. Ozone and hydrogen peroxide elimination filter
5. Ozone generator
60. Ozone supplying tube
61. Ozone sprayer
7. Control device
8. UV lamp
9. Sensor unit

### BEST MODE FOR CARRYING OUT THE INVENTION

An embodiment of the present invention in an isolator is to be described in detail below with reference to the drawings.

### [EMBODIMENT 1]

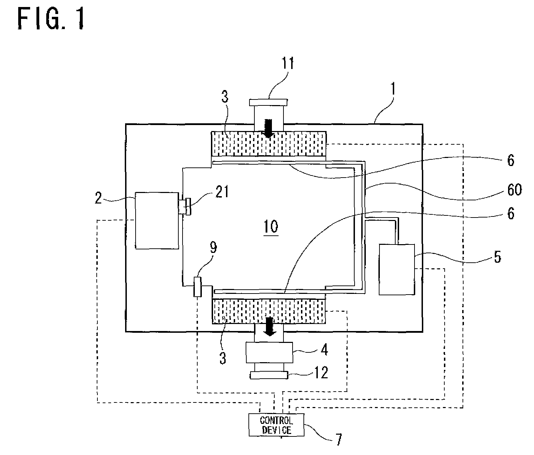

In a first isolator according to the present invention, as shown in FIG. 1, a sterile room 10 having an inlet opening 11 and an outlet opening 12 is formed in a cabinet 1. In the sterile room 10, HEPA filters 3, 3 are placed so as to cover the inlet opening 11 and the outlet opening 12 each.

In the sterile room 10, ozone spraying devices 6, 6 are placed opposite to both the HEPA filters 3, 3. The ozone spraying devices 6, 6 are connected to an ozone generator 5 (sterilizing material generating part) via an ozone supplying tube 60 (detoxifying material supplying part) for supplying ozone.

Also, to the sterile room 10, connected is a hydrogen peroxide supplying tube 21 for supplying hydrogen peroxide which is a sterilizing material from a hydrogen peroxide generator 2. An ozone and hydrogen peroxide eliminating filter 4 consisting of activated carbon or the like is placed between the outlet opening 12 and the HEPA filter 3.

Further, the sterile room 10 is provided with a sensor unit 9 detecting temperature, humidity, hydrogen peroxide concentration, ozone concentration and the like. A detection signal is supplied to a control device 7. The hydrogen peroxide generator 2, the ozone generator 5 and the like are controlled by the control device 7.

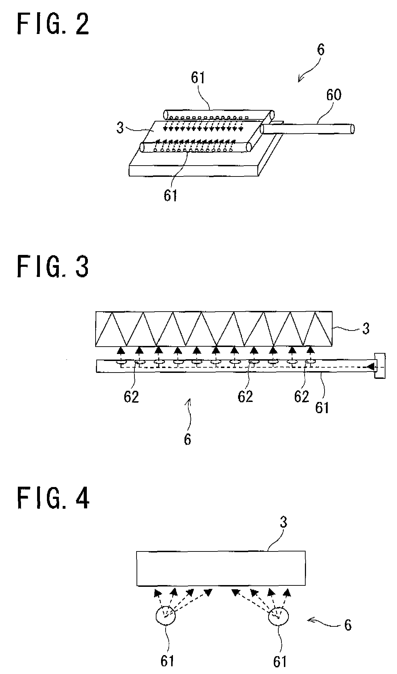

An ozone spraying device 6 comprises a pair of ozone sprayers 61, 61 which branches from the ozone supplying tube 60 as shown in FIG. 2. In the ozone sprayer 61, a plurality of nozzles 62 opens toward the HEPA filter 3 as shown in FIG. 3.

From the pair of ozone sprayers 61, 61 toward a whole surface of the HEPA filter 3, sprayed is mist (hereinafter referred to as ozone mist) of a liquid including ozone which is a detoxifying material for detoxifying the sterilizing material as shown in FIG. 4.

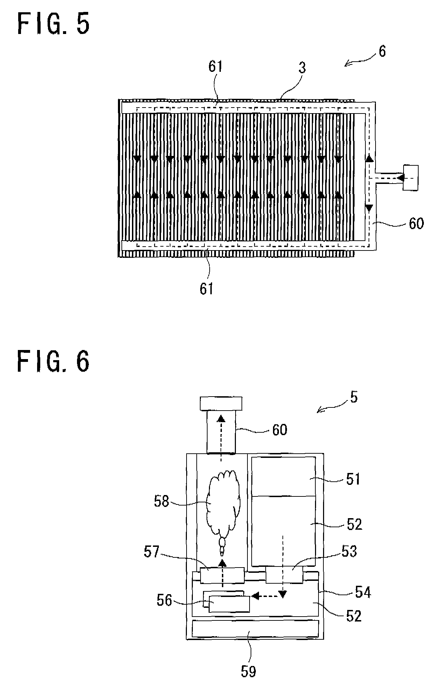

As shown FIG. 5, the pair of ozone sprayers 61, 61 is placed at both end parts of the HEPA filter 3. Each of the ozone sprayers 61 extends in a direction perpendicular to a plurality of fold lines of the HEPA filter 3. Therefore, ozone gas or ozone mist is sprayed from the both ozone sprayers 61, 61 along the fold lines of the HEPA filter 3, thereby supplying ozone to every corner of the HEPA filter 3.

The ozone generator 5 may be formed by an ozone mist generator shown in FIG. 6. In this ozone mist generator, under the control of a control board 59, ozone mist 58 is generated by supplying pure water 52 in a pure water tank 51 from a water seal cap 53 into an electrolysis tank 54, generating ozone water by an ozone generating electrode 56 in the electrolysis tank 54, and giving ultrasonic oscillation to the ozone water by an ultrasonic oscillator 57. The ozone mist 58 is supplied from the ozone supplying tube 60 to the outside.

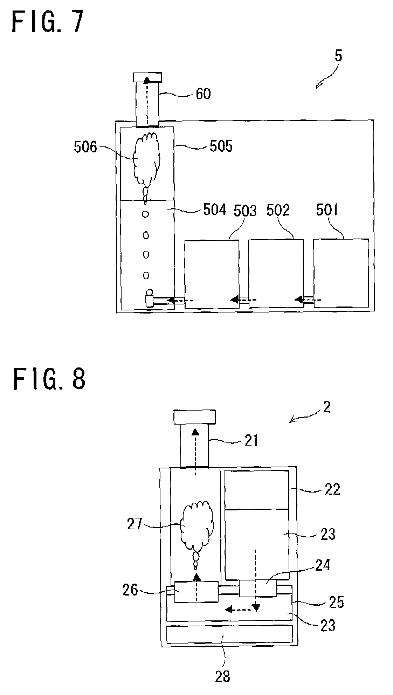

Also, the ozone generator 5 may be formed by an ozone gas generator shown in FIG. 7. In this ozone gas generator, oxygen is supplied from an oxygen supply source 501 via a supplier 502 to a discharge ozone generator 503. Ozone gas is generated by the discharge in the discharge ozone generator 503, and the ozone gas is supplied to a humidifier 505 to be humidified by pure water 504, thereby obtaining humidified ozone 506.

The hydrogen peroxide generator 2 may be formed by a hydrogen peroxide mist generator shown in FIG. 8. In this hydrogen peroxide mist generator, under the control of a control board 28, hydrogen peroxide mist 27 is generated by supplying hydrogen peroxide water 23 in a hydrogen peroxide water tank 22 from a water seal cap 24 into a hydrogen peroxide water tank 25, and giving ultrasonic oscillation to the hydrogen peroxide water by an ultrasonic oscillator 26.

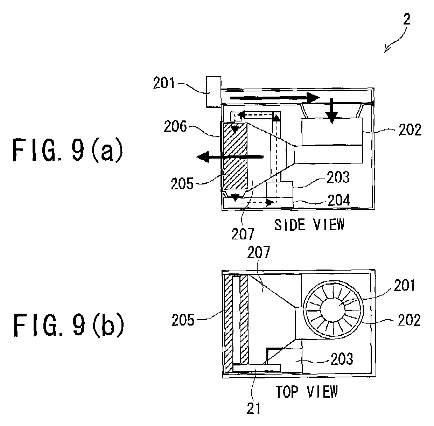

The hydrogen peroxide generator 2 may be formed by a hydrogen peroxide vaporizer shown in FIGS. 9. In this hydrogen peroxide vaporizer, air taken in from an inlet opening 201 to a blowing duct 207 by operation of a blowing fan 202 is eliminated via an element 205 placed at an outlet of the blowing duct 207 from an exhaust opening 206, while hydrogen peroxide water is pumped up by a pump 203 from a hydrogen peroxide water tank 204 to be dropped to the element 205, and thereby hydrogen peroxide water is vaporized by air current passing through the element 205. And then, vaporized hydrogen peroxide gas is supplied from the hydrogen peroxide supplying tube 21 to the outside.

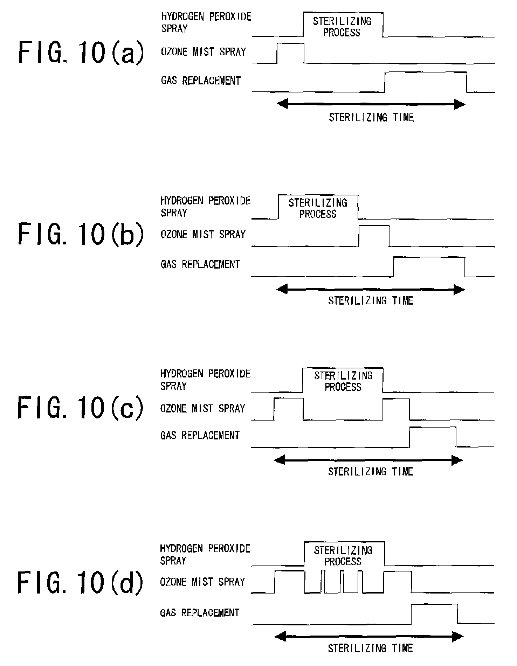

In the isolator described above, a process shown in FIG. 10a is performed in order to sterilize the sterile room 10. As shown in the figure, before the sterilization process in which hydrogen peroxide gas or mist is sprayed from the hydrogen peroxide generator 2 to the sterile room 10, ozone mist or ozone gas is sprayed from the ozone spraying devices 6. And, then, after the sterilization process, the conventional gas replacement is performed.

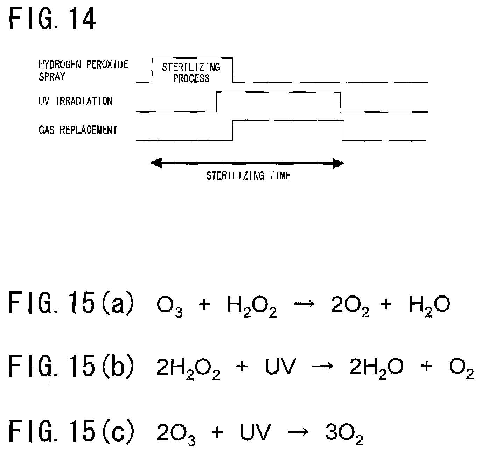

Thus, by spraying the ozone mist or ozone gas from the ozone spraying devices 6 before the sterilization process of sterilizing the sterile room 10, ozone is attached to the HEPA filter 3, and hydrogen peroxide is added to the ozone attached to the HEPA filter 3 in the sterilization process thereafter. As a result, as shown in FIG. 15a, ozone and hydrogen peroxide react to generate oxygen and water, thereby performing the detoxifying process on hydrogen peroxide.

Therefore, the gas replacement after the sterilization process ends in a short time as shown in FIG. 10a, thereby considerably shortening time required for the sterilization.

Also, in the isolator described above, it is possible that the ozone mist or ozone gas is sprayed from the ozone spraying devices 6 after the sterilization process as shown in FIG. 10b, and thereafter the conventional gas replacement process is performed.

Thus, also by spraying the ozone mist or ozone gas only after the sterilization process, ozone is added to the hydrogen peroxide attached to the HEPA filter 3 in the sterilization process. As a result, hydrogen peroxide and ozone react to generate oxygen and water.

Therefore, the gas replacement ends in a short time as shown in FIG. 10b, thereby considerably shortening the time required for the sterilization.

Also, in the isolator described above, it is possible that the ozone mist or ozone gas is sprayed from the ozone spraying devices 6 before and after the sterilization process as shown in FIG. 10c, and thereafter the conventional gas replacement is performed.

Time for the gas replacement is thereby further shortened.

Further, in the isolator described above, it is possible that the ozone mist or ozone gas is sprayed before and after the sterilization process and also once or a plurality of times during the sterilization process as shown in FIG. 10d, and thereafter the conventional gas replacement is performed.

Time for the gas replacement is further shortened, thereby considerably shortening time required for the sterilization.

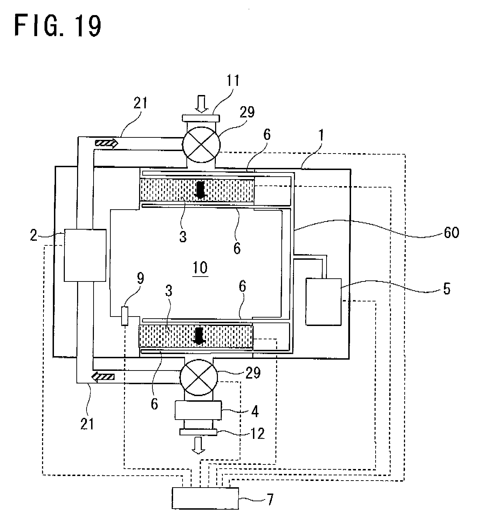

Also, in the isolator described above, the hydrogen peroxide supplying tube 21 supplying hydrogen peroxide generated in the hydrogen peroxide generator 2 is directly connected to the sterile room 10. However, it is also possible to perform in a circulation sterilization type, as shown in FIG. 19, in which the hydrogen peroxide generator 2 is placed outside of the sterile room 10, and hydrogen peroxide is circulated in an order of the hydrogen peroxide generator 2, the inlet opening side HEPA filter 3, the sterile room 10, the outlet opening side HEPA filter 3, and the hydrogen peroxide generator 2 to sterilize the sterile room 10.

In such a case, as shown in FIG. 19, the ozone supplying tube 60 is placed on each of four surfaces: the sterile room side surface, the sterile room side surface of the inlet opening side HEPA filter 3, the sterile room side surface, the outlet opening side surface of the outlet side HEPA filter 3, to perform the detoxifying process by spraying ozone. Ozone is not necessarily sprayed to all of the four surfaces, but may be sprayed depending on the degree of the attachment of hydrogen peroxide to the HEPA filters 3, 3. The attachment amount of hydrogen peroxide to the HEPA filters 3, 3 is greatest on the inlet opening side surface of the inlet opening side HEPA filter 3, followed by the sterile room side surface of the inlet opening side HEPA filter 3, the sterile room side surface, and outlet opening side surface of the outlet side HEPA filter 3 in this order.

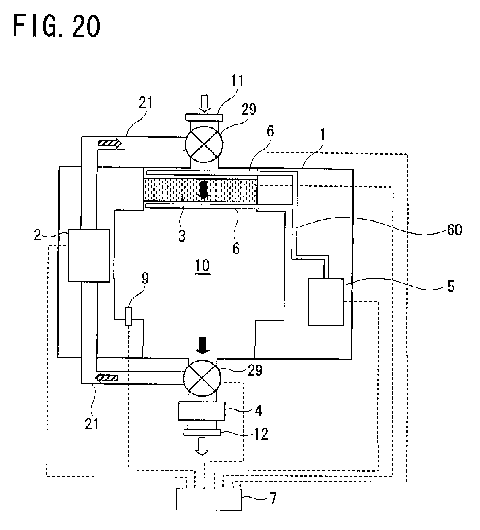

Also, as shown in FIG. 20, this is also possible in a similar manner in the isolator in which the HEPA filter 3 is placed only at the inlet opening.

In the isolator described above, used are hydrogen peroxide (first active oxygen species) as a sterilizing material and ozone (second active oxygen species) as a detoxifying material. The active oxygen species includes hydroxyl radical having the strongest oxidation effect followed, in the order, by ozone, hypochlorous acid, hydrogen peroxide, superoxide, singlet oxygen and the like. Such a combination that the second active oxygen species has greater oxidizing power than the first active oxygen species is preferable. However, the opposite combination is also possible. For example, it is possible to use ozone (first active oxygen species) as the sterilizing material and hydrogen peroxide (second active oxygen species) as the detoxifying material. In such a case, although ozone has a greater oxidizing power than hydrogen peroxide, ozone and hydrogen peroxide react as shown in FIG. 15a to generate oxygen and water, thereby performing the detoxifying process against ozone.

In the case of the combination of ozone and hydrogen peroxide, not only they react with each other to obtain an effect of shortening the time required for sterilisation, but also expected is a more effective sterilizing activity due to a strong oxidizing power of hydroxyl radical or the like which is generated in the process before ozone and hydrogen peroxide react to generate oxygen and water.

Also, in the case where the sterilizing material is hydrogen peroxide or ozone and the detoxifying material is alkaline water, hydroxide ion in the alkaline water and the sterilizing material come into contact with each other, thereby realizing decomposition.

### [EMBODIMENT 2]

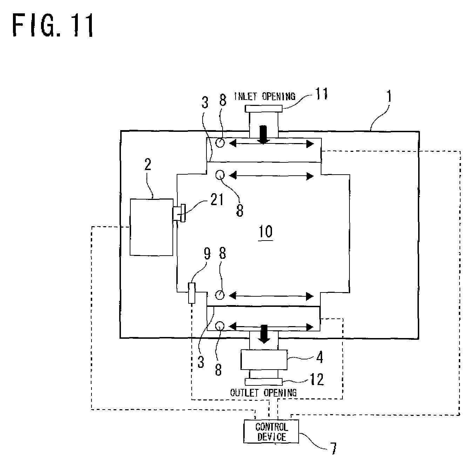

In a second isolator according to the present invention, as shown in FIG. 11, a sterile room 10 having an inlet opening 11 and an outlet opening 12 is formed in a cabinet 1. In the sterile room 10, HEPA filters 3, 3 are placed so as to cover the inlet opening 11 and the outlet opening 12 each.

In the sterile room 10, two UV lamps 8, 8 are provided on both sides of each HEPA filter 3.

Also, to the sterile room 10, the hydrogen peroxide supplying tube 21 of the hydrogen peroxide generator 2 is connected. An ozone and hydrogen peroxide eliminating filter 4 consisting of activated carbon or the like is placed between the outlet opening 12 and the HEPA filter 3.

Further, the sterile room 10 is provided with the sensor unit 9 detecting temperature, humidity, hydrogen peroxide concentration and the like. The detection signal is supplied to a control device 7, and the hydrogen peroxide generator 2, the UV lamp 8 and the like are controlled by the control device 7.

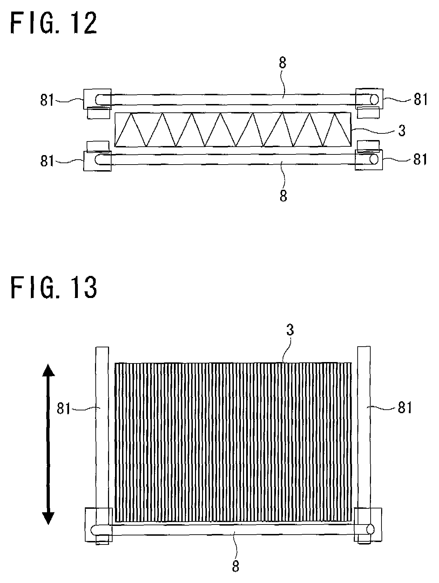

As shown in FIGS. 12 and 13, the two UV lamps 8, 8 provided above and under the HEPA filter 3 each has a length somewhat greater than a width of the HEPA filter 3, and has both end parts supported by reciprocating transfer mechanisms 81, 81.

Both UV lamps 8, 8 reciprocate along both surfaces of the HEPA filter 3 by means of drive of the reciprocating transfer mechanisms 81, 81, and irradiate ultraviolet toward both surfaces of the HEPA filter 3.

In the isolator described above, a process shown in FIG. 14 is performed in order to sterilize the sterile room 10. As shown in the figure, first, hydrogen peroxide gas or mist is sprayed from the hydrogen peroxide generator 2 to the sterile room 10 to start the sterilization process which sterilizes the sterile room 10, and then, just before or just after the end of the process, all the UV lamps 8 are lighted, the UV lamps 8 reciprocate, and ultraviolet is irradiated to both surfaces of the HEPA filter 3.

And then, after the end of the sterilization process, the conventional gas replacement is started. And thereafter, the ultraviolet irradiation and the gas replacement end.

Thus, after starting the sterilization process, by irradiating ultraviolet on both surfaces of the HEPA filter 3 from the UV lamp 8, the ultraviolet acts on the hydrogen peroxide attached to the HEPA filter 3. As a result, as shown in FIG. 15b, hydrogen peroxide is subjected to the action of ultraviolet to generate oxygen and water, thereby performing the detoxifying process on hydrogen peroxide.

Therefore, the gas replacement after the sterilization process ends in a short time as shown in FIG. 10a, thereby considerably shortening the time required for the sterilization.

This is also possible in the sterilization circulation type isolator as shown in FIG. 19, and the irradiation may be performed depending on the degree of the attachment of hydrogen peroxide to the HEPA filters 3, 3. The attachment amount of hydrogen peroxide to the HEPA filters 3, 3 is the greatest on the inlet opening side surface of the inlet opening side HEPA, filter 3, followed, in the order, by the sterile room side surface of the inlet opening side HEPA filter 3, the sterile room side surface, and outlet opening side surface of the outlet side HEPA filter 3. Therefore, it is preferable to perform detoxifying depending on the attachment amount. Further, it is preferable to preferentially detoxify the inlet opening side surface.

Also, as shown in FIG. 20, this is also possible in a similar manner in the isolator in which the HEPA filter 3 is placed only at the inlet opening.

In the isolator described above, the ultraviolet irradiation device (irradiation means) is used. However, an ultrasound irradiation device, an infrared irradiation device, and a heat irradiation device, or any combination thereof may also be used. Ultraviolet irradiation has the highest effect of decomposition, followed by ultrasound irradiation, heat irradiation, and infrared irradiation. This is because, in the case of ultraviolet irradiation, ultraviolet is directly absorbed by a sterilizing material molecule, and the absorption energy cuts interatomic bond, and therefore, the efficiency is good. In the case of ultrasound irradiation, thermal decomposition reaction generated in cavitation (ultrahigh-temperature and ultrahigh-pressure foam having a diameter of several nanometers) in the solution cuts the interatomic bond of the sterilizing material. However, it concurrently acts on the interatomic bond of the molecule of a solvent in which the sterilizing material is dissolved, and therefore, the efficiency is bad. Also, in the case of heat irradiation (such as a heater and the like) and infrared irradiation, the given heat energy is first consumed in evaporation of the sterilizing material and the solvent, and then the heat energy is later consumed in decomposition of the sterilizing material, and therefore, the efficiency is bad.

In the case where the ultrasound is irradiated to the HEPA filter using the ultrasound irradiation device, the sterilizing material is detoxified thorough thermal decomposition by heat generated by local vibration on the filter. Further, the vibration may be generated more effectively in a structure in which the HEPA filter covers a net plate member having openings like a mesh formed by a material that can endure exposure of sterilizing material.

In the isolator described above, hydrogen peroxide is used as the sterilizing material. However, ozone may be used as the sterilizing material. In such a case too, as shown in FIG. 15c, ozone is subjected to the action of ultraviolet to generate oxygen and water, thereby performing the detoxifying process on ozone.

Also, in the isolator described above, not only ozone or hydrogen peroxide is subjected to the action of ultraviolet to obtain an effect of shortening the time required for sterilization, but also expected is a more effective sterilizing activity due to a strong oxidizing power of hydroxyl radical or the like which is generated in the process before ozone or hydrogen peroxide is subjected to the action of ultraviolet to be decomposed and generate oxygen.

### [EMBODIMENT 3]

In a third isolator of the present invention, as shown in FIG. 16, a sterile room 10 having an inlet opening 11 and an outlet opening 12 is formed in a cabinet 1. In the sterile room 10, HEPA filters 3, 3 are placed so as to cover the inlet opening 11 and the outlet opening 12 each. Unlike conventional isolators, platinum is included in a surface of the HEPA filter by sputtering in advance.

Also, to the sterile room 10, connected is a hydrogen peroxide supplying tube 21 for supplying hydrogen peroxide which is a sterilizing material from a hydrogen peroxide generator 2. An ozone and hydrogen peroxide eliminating filter 4 consisting of activated carbon or the like is placed between the outlet opening 12 and the HEPA filter 3.

In the isolator described above, in order to sterilize the sterile room 10, hydrogen peroxide gas or mist is strayed in the sterile room 10 from the hydrogen peroxide generator 2 to sterilize the sterile room 10, hydrogen peroxide attached to the HEPA filter 3 is decomposed and eliminated by catalytic reaction of platinum included in the HEPA filter.

In the isolator described above, platinum is used as a detoxifying material included in the HEPA filter 3. However, it may be a material including one or more of oxidant, reducing agent, activated carbon, platinum, ferric oxide, copper oxide, manganese, catalase, fullerene, zinc complex of porphyrine, and compound of chlorophyll class.

Platinum is excellent in reusability and catalytic efficiency among the detoxifying materials, followed by oxidant, reducing agent, activated carbon, ferric oxide, copper oxide, manganese, fullerene, and zinc complex of porphyrine in this order. Catalase is effective in the case where the sterilizing material is hydrogen peroxide.

Especially, zinc complex of porphyrine and compound of chlorophyll class generate an enzyme radical on a surface of the complex when light having a wavelength of around 400 nm is irradiated, and the sterilizing material is detoxified by this enzyme radical. Therefore, it is preferable to provide a light source which irradiates the light having a wavelength of around 400 nm.

And then, when the conventional gas replacement is performed after the sterilization process, the gas replacement after the sterilization process ends in a short time due to decomposing action of the detoxifying material against the sterilizing material, thereby considerably shortening the time required for the sterilization.

This is also possible in a similar manner, as shown in FIG. 20, in the isolator in which the HEPA filter 3 is placed only at the inlet opening.

The present invention is not limited to the foregoing embodiment in construction but can be modified variously within the technical scope set forth in the claims. Also, the sterile environment maintaining apparatus according to the present invention may be adopted not only in the isolators but also in various devices having a sterile room such as a clean bench and the like.

## Claims

1. A sterile environment maintaining apparatus comprising a sterile room having an inlet opening and an outlet opening, a fine particle capture filter placed so as to cover the inlet opening or the inlet opening and the outlet opening of the sterile room, and a sterilizing material supplying part supplying a sterilizing material to the sterile room, wherein the sterile environment maintaining apparatus is provided with a detoxifying material supplying part releasing a detoxifying material for detoxifying the sterilizing material toward the fine particle capture filter.

2. The sterile environment maintaining apparatus according to claim 1, wherein the detoxifying material supplying part releases the detoxifying material toward an inlet opening side surface of the fine particle capture filter placed so as to cover the inlet opening.

3. The sterile environment maintaining apparatus according to claim 1 or 2, wherein the sterilizing material supplying part supplies gas or liquid fine particle including the sterilizing material to the sterile room, and the detoxifying material supplying part supplies gas or liquid fine particle including the detoxifying material toward the fine particle capture filter.

4. The sterile environment maintaining apparatus according to any one of claims 1 to 3, wherein the sterilizing material is a first active oxygen species, and the detoxifying material is a second active oxygen species.

5. The sterile environment maintaining apparatus according to any one of claims 1 to 4, wherein the sterilizing material is hydrogen peroxide or ozone, and the detoxifying material is alkaline water.

6. A sterile environment maintaining apparatus comprising a sterile room having an inlet opening and an outlet opening, a fine particle capture filter placed so as to cover the inlet opening or the inlet opening and the outlet opening of the sterile room, and a sterilizing material supplying part supplying a sterilizing material to the sterile room, wherein the sterile environment maintaining apparatus is provided with irradiating means for detoxifying the sterilizing material toward, the fine particle capture filter.

7. The sterile environment maintaining apparatus according to claim 6, wherein, the sterilizing material is an active oxygen species, and the irradiating means is either of an ultraviolet irradiation device, an ultrasound irradiation device, a heat irradiation device or an infrared irradiation device, or any combination thereof.

8. A sterile environment maintaining apparatus comprising a sterile room having an inlet opening and an outlet opening, a fine particle capture filter placed so as to cover the inlet opening and the outlet opening of the sterile room, and a sterilizing material supplying part supplying a sterilizing material to the sterile room, wherein the fine particle capture filter includes a detoxifying material for detoxifying the sterilizing material.

9. The sterile environment maintaining apparatus according to claim 8, wherein the detoxifying material is a material including one or more of oxidant, reducing agent, activated carbon, platinum, ferric oxide, copper oxide, manganese oxide, catalase, fullerene, zinc complex of porphyrine, and compound of chlorophyll class.
